# EUROPEAN PATENT SPECIFICATION

(11) **EP 0 670 827 B1**
(45) Date of publication and mention of the grant of the patent: **16.04.1997**
(21) Application number: 94900807.2
(22) Date of filing: 11.11.1993
(51) Int. Cl.: C07C 235/06, C07C 235/08, C11D 1/66, A61K 7/50, A61K 7/24, A61K 7/15

(54) **ALKYLGLYCERAMIDES AND THEIR USE AS SURFACTANTS**
ALKYLGLYCERINSÄUREAMIDE UND DEREN VERWENDUNG ALS GRENZFLÄCHENAKTIVE SUBSTANZEN
ALKYLGLYCERAMIDES ET LEUR UTILISATION COMME TENSIOACTIFS

(30) Priority: 25.11.1992 US 981972
(43) Date of publication of application: 13.09.1995
(73) Proprietor: UNILEVER PLC, London EC4P 4BQ (GB); UNILEVER N.V., 3013 AL Rotterdam (NL)
(72) Inventor: RAHMAN, Mohammad, Abdur 159 Bogert Road, Bergen County, NJ 07661 (US); WU, Shang-Ren, Mahwah, NJ 07430 (US); GARRETT, Peter, Robert Charlesville, Pantymwyn Clwyd CH7 5EH (GB)
(74) Representative: Fransella, Mary Evelyn
(86) International application number: PCT/EP93/03172
(87) International publication number: WO 94/12467

(56) References cited:
- FR-A- 2 523 962
- CHEMICAL ABSTRACTS, vol. 110, no. 1, 2 January 1989, Columbus, Ohio, USA, T. MASE et al, "Saturated heterocyclic carboxamides, especially thiazolidinecarboxamides, useful as PAF antagonists, their pharmaceutical compositions, and processes and intermediates for their preparation" page 552, abstract no. 23877v; & EP-A-0 279 681

## Description

The present invention relates to novel surface-active N-alkylglyceramide compounds and to their use as surface-active and/or foaming agents, for example, in detergent or personal product compositions. The compounds may advantageously be used in combination with other surfactants to give enhanced foaming and detergency.

### BACKGROUND AND PRIOR ART

Glyceric acid, CH₂OH-CHOH-COOH, is a readily available material. It would therefore be a great advantage to design a surfactant based on the material, particularly a surfactant which can enhance and stabilise foam. Unexpectedly, it has been found that the N-alkylglyceramides of the invention, especially when used in combination with other surfactants, meet this criterion.

The ccmpounds of the invention have the formula CH₂OH-CHOH-CO-NHR, where R is a hydrocarbon group (defined below). There are compounds disclosed in the art which have some structural similarities to the alkylglyceramides of the invention.

N-alkanolamides, for example, form a well-known class of compounds which have, of course, both an alcohol and an amide functional group. These compounds both enhance and stabilise foam. However, these compounds are structurally different from the N-alkylglyceramides of the invention, in that the hydroxylic group is attached directly to the amine nitrogen atom rather than via the carbonyl carbon atom.

US 5 009 814 (Kelkenberg et al/Hüls) teaches N-polyhydroxyalkyl fatty acid amide compounds having the formula 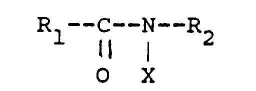 wherein R₁ is alkyl, R₂ is hydrogen, an alkyl group or an alkylene oxide group and X is a polyhydroxyalkyl group. Again, in these compounds, the polyhydroxyalkyl group is attached to the nitrogen atom rather than to the carbonyl group.

FR 2 523 962A (Centre Nationale de Recherche/Monsigny) teaches amides having the formula

CH₂OH-(CHOH)ₘ-CO-NHR

in which m is 2 to 6 and R is a linear or branched alkyl group having 6 to 18 carbon atoms. In these compounds, m must equal at least 2 and, therefore, these are not amides of glyceric acid. Also, there is also no teaching or suggestion that alkylamides may be used as cosurfactants with other detergent-active compounds.

Our copending European Patent Application EP 550 277A, published on 7 July 1993, having an application date of 30 December 1992 and a priority date of 31 December 1991, describes and claims alkylglycerates (alkyl esters of glyceric acid) and their use as surfactants and cosurfactants in detergent compositions.

### DEFINITION OF THE INVENTION

The present invention provides a surface-active and/or foaming and/or foam enhancing N-alkylglyceramide or N-alkenylglyceramide of the formula I: 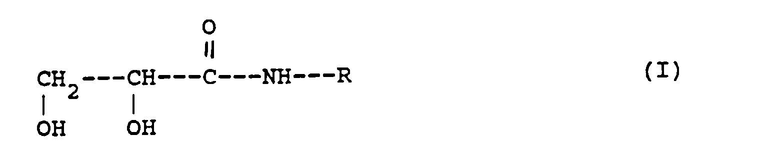 wherein R is a straight- or branched chain alkyl or alkenyl group containing from 8 to 24 carbon atoms.

The invention further provides the use of a compound as defined above - an N-alkylglyceramide - as a surfactant and/or foaming agent and/or foam enhancer in a surface-active and/or foaming composition.

The invention also provides a detergent or personal product composition comprising a surface-active and/or foaming and/or foam-enhancing amount of a compound as defined above.

### DETAILED DESCRIPTION OF THE INVENTION

### The compounds and their preparation

In the formula I, R is a straight- or branched-chain alkyl or alkenyl group having 8 to 24 aliphatic carbon atoms.

Especially preferred are linear and branched C₈ to C₁₆ alkyl groups, for example, a linear C₈ or C₁₀ alkyl group.

The compounds of the invention have surface-active and/or foaming and/or foam-enhancing properties. Depending on chain length, some compounds will be especially useful as surfactants in their own right, while others will be most valuable as cosurfactants or foam enhancers when used in combination with other surfactants: generally in significant amounts when used as cosurfactants, and in relatively small amounts when used as foam enhancers.

The novel surfactants of the present invention are based on a readily available material, glyceric acid. They may be prepared by reacting together a lower (eg C₁₋₄) ester of glyceric acid, for example, methyl glycerate, with an amine of the formula RNH, where R is as defined previously. This may be carried out, for example, in anhydrous methanol at a moderately elevated temperature under an atmosphere of nitrogen.

### Detergent compositions

The novel surfactants of the present invention may be incorporated in detergent compositions of all physical types, for example, powders, liquids, gels and solid bars.

A detergent composition in accordance with the invention may suitably contain from 5 to 70 wt% of a surfactant system which includes or consists of an N-alkylglyceramide in accordance with the invention. The amount of N-alkylglyceramide in the composition may suitably range from 1 to 70 wt%, the lower end of the range representing compositions in which the N-alkylglyceramide is functioning as a foam enhancer to another surfactant present in a much larger amount.

### N-Alkylglyceramides as cosurfactants

Especially preferred are detergent compositions containing a surfactant system comprising from 5 to 95 wt% of an N-alkylglyceramide of the invention and from 5 to 95 wt% of one or more other surfactants selected from soap, anionic surfactants, nonionic surfactants, cationic surfactants, amphoteric surfactant and zwitterionic surfactants.

The N-alkylglyceramide of the invention have been found to enhance foam stability when used as a cosurfactant, particularly when comprising from 25 to 90 wt%, more preferably from 40 to 80 wt%, of the surfactant system.

The other surfactant may be chosen from the many suitable detergent-active compounds available. These are fully described in the literature, for example, in "Surface-Active Agents and Detergents", Volumes I and II, by Schwartz, Perry and Berch.

The preferred surfactants that can be used are soaps and synthetic non-soap anionic and nonionic compounds; especially preferred are non-soap anionic surfactants.

Anionic surfactants are well-known to those skilled in the art. Examples include alkylbenzene sulphonates, particularly linear alkylbenzene sulphonates having an alkyl chain length of C₈-C₁₅; primary and secondary alkyl sulphates, particularly C₁₂-C₁₅ primary alkyl sulphates; alkyl ether sulphates; olefin sulphonates; alkyl xylene sulphonates; dialkyl sulphosuccinates; and fatty acid ester sulphonates. Sodium salts are generally preferred.

Nonionic surfactants that may be used include the primary and secondary alcohol ethoxylates, especially the C₈₋C₂₀ aliphatic alcohols ethoxylated with an average of from 1 to 20 moles of ethylene oxide per mole of alcohol, and more especially the C₁₀₋C₁₅ primary and secondary aliphatic alcohols ethoxylated with an average of from 1 to 10 moles of ethylene oxide per mole of alcohol. Non-ethoxylated nonionic surfactants include alkylpolyglycosides, glycerol monoethers, and polyhydroxyamides (glucamide).

Especially preferred surfactants for use in combination with the N-alkylglyceramides of the invention are non-soap surfactants, more especially anionic sulphonate or sulphate-type surfactants, notably alkylbenzene sulphonates and primary alcohol sulphates.

### N-alkylglyceramides as foam enhancers

The N-alkylglyceramides are also useful for incorporation in relatively small amounts, as foam enhancers, in high-foaming detergent compositions, for example, those intended for washing fabrics by hand. Such compositions typically contain a relatively high level of anionic surfactant, for example, alkylbenzene sulphonate or primary alcohol sulphate.

The foaming of anionic surfactants such as alkylbenzene sulphonate can be quite badly affected by the presence of fatty soil which acts as an antifoam. The presence of quite small amounts of N-alkylglyceramide, eg 5-10 mole% based on the anionic surfactant, has been found to counteract this loss of foaming capacity successfully.

### Other ingredients

Detergent compositions of the invention may also, depending on the purpose for which they have been formulated, contain various ancillary ingredients. For example, fabric washing compositions may contain detergency builders such as phosphates, aluminosilicates, polycarboxylate polymers, citrate; bleach components such as sodium perborate or percarbonate, bleach activators and bleach stabilisers; sodium carbonate; sodium silicate; antiredeposition agents such as cellulosic polymers; fluorescers; inorganic salts such as sodium sulphate; proteolytic and lipolytic enzymes; dyes; coloured speckles; perfumes; fabric softening compounds. This list is not intended to be exhaustive.

Table 1 below shows a typical outline formulation.

**Table 1:**

| heavy duty fabric washing powder | |
|---|---|
| Ingredient | Weight percentage range |
| Surfactant | 5 to 70 wt%, preferably 5 to 40 wt% |
| Builder | 5 to 80 wt%, preferably 10 to 60 wt% |
| Persalt bleach | 5 to 35 wt%, preferably 10 to 25 wt% |
| Bleach activator | 1 to 8 wt%, preferably 2 to 5 wt% |
| Sodium carbonate | 0 to 60 wt%, preferably 2 to 40 wt% |
| Sodium silicate | 0 to 15 wt% |
| Fatty acid soap | 0 to 5 wt%. |
| Optional minor ingredients, salts and water. | |

The optional minor ingredients may include antiredeposition agents such as cellulosic polymers; fluorescers; inorganic salts such as sodium sulphate; lather control agents or lather boosters as appropriate; proteolytic and lipolytic enzymes; dyes; coloured speckles; perfumes; foam controllers; and fabric softening compounds. This list is not intended to be exhaustive.

The N-alkylglyceramides are also suitable for incorporation into aqueous (both isotropic and lamellar) and non-aqueous liquid detergent compositions for washing fabrics. Suitable formulations are disclosed, for example, in US 4 244 840, EP 38 101A, EP 160 342A and EP 140 452A. Structured lamellar liquids may contain a deflocculating polymer as described and claimed in EP 346 995A (Unilever).

The compositions of the invention are also suitable for incorporation into light-duty (non-bleaching, and generally unbuilt) high-foaming liquid detergent compositions, for example, those used for hand washing of dishes or of delicate fabrics.

### Other compositions

The compounds of the invention are also suitable for other applications in which surfactant and/or foaming and/or foam-enhancing properties are useful; in particular, for personal product (personal care) formulations of various types.

Personal product compositions of the invention may be, for example, toilet bar compositions, facial or body cleansing compositions, shampoos for hair or body, conditioners or conditioning shampoos, shower gels, cosmetic compositions, shaving preparations, or dental care compositions.

### Toilet bar compositions

Typical toilet bar compositions are those comprising fatty acid soaps used in combination with a detergent other than fatty acid soap and free fatty acids; however, the composition may comprise no fatty acid soap and may be based on actives other than fatty acid soap. Mildness-improving salts, such as alkali metal salts of isethionate, are also typically added. In addition other ingredients, such as germicides, perfumes, colorants, pigments, suds-boosting salts and anti-mushing agents may also be added.

A typical outline formulation might be as shown in Table 2 below.

### Facial or body cleansing compositions

N-alkylglyceramides may also be present in a facial or body cleansing composition. Examples of such cleaning compositions are described, for example, in US 4 812 253 (Small et al) and US 4 526 710 (Fujisawa).

Typically, cleansing compositions will comprise a fatty acid soap together with a non-soap surfactant, preferably a mild synthetic surfactant; a moisturiser or emollient; and polymeric skin feel and mildness aids. The compositions may further optionally include thickener (eg magnesium aluminium silicate, Carbopol (Trade Mark), water soluble polymers (eg carboxymethylcellulose), dyes, hydrotropes, brighteners, perfumes and germicides.

A typical outline formulation might be as in Table 3 below.

### Shampoo compositions

The N-alkylglyceramide surfactant of the invention may be used, for example, in a hair or body shampoo. Examples of such compositions are described in US 4 854 333 (Inman) and US 4 526 710 (Fujisawa).

Shampoo compositions typically comprise a further surfactant; a compound for treating dandruff, eg selenium sulphide; a suspending agent; a thickening agent, eg cross-linked polyacrylate; a nonionic polymer; a nonvolatile silicone fluid; a preservative; a cationic surfactant; a pH adjusting agent; a perfume; a dye; a sequestering agent, such as disodium ethylenediamine tetraacetate.

A typical outline shampoo composition might be as in Table 4 below.

### Toothpaste compositions

In yet another embodiment of the invention, the surfactant may be used in a toothpaste composition such as is taught and is described in US 4 935 227 (Duckworth).

Such compositions generally comprise abrasive gels (eg calcium carbonate), oral therapeutic agents (for example, fluorine containing compound), cosurfactants, flavouring agents, sweetening agents, humectants and binding or thickening gels.

A typical outline formulation might be as shown in Table 5 below.

**Table 2:**

| toilet bar composition | |
|---|---|
| Ingredient | Weight % |
| C₈ to C₂₄ fatty acid | 5-60 |
| N-alkylglyceramide | 1-45 |
| Cosurfactant other than N-alkylglyceramide | 0-50 |
| Alkyl or aryl sulphate or sulphonate | 0-5 |
| Moisturiser (eg sorbitol or glycerol) | 0.1-10 |
| Water soluble polymer eg cellulose or polyacrylate) | 0-10 |
| Sequestering agent eg citrate | 0.1-0.5 |
| Dyestuff | <0.1 |
| Optical brightener | <0.1 |
| Whitening agent | 0.1-0.4 |
| Fragrance | 0.1-2.0 |
| Water | Balance |

**Table 3:**

| facial or body cleansing composition | |
|---|---|
| Ingredient | Weight % |
| C₈₋₂₄ fatty acid salt (eg triethanolamine salt) | 1-45 |
| N-alkylglyceramide | 10-75 |
| Alkyl or aryl sulphate or sulphonate | 0-20 |
| Cosurfactant (eg cocoamidobetaine) | 1-15 |
| Moisturiser (eg sorbitol) | 0.1-15 |
| Refattying alcohol | 0.5-5 |
| Water soluble polymer | 0-10 |
| Thickener | 0-15 |
| Conditioner (eg quaternised cellulose) | 0.1-15 |
| Sequestering agents (eg citrate) | 0.1-0.4 |
| Dyestuff | <0.1 |
| Optical brighteners | <0.1 |
| Whitening agents | 0.1-0.4 |
| Fragrance | 0.1-3.0 |
| Preservatives | 0-0.2 |
| Water | Balance |

**Table 4:**

| shampoo composition | |
|---|---|
| Ingredient | Weight % |
| N-alkylglyceramide | 5-15 |
| Anionic surfactant | 0-10 |
| Amphoteric surfactant | 0-10 |
| Lauric monoethanolamide | 0-5 |
| Thickener | 0-5 |
| Fragrance | 0-2 |
| Preservative | 0-1 |
| Water | Balance |

**Table 5:**

| toothpaste composition | |
|---|---|
| Ingredient | Weight % |
| Synthetic surfactant (eg sodium lauryl sulphate) | 1.5 |
| N-alkylglyceramide | 1-10 |
| Alkyl or aryl sulphate or sulphonate | 0-1 |
| Abrasive (eg silicic acid/CaCO₃) | 20-55 |
| Active ingredients (eg pyrophosphate) | 0.1-2 |
| Humectant (eg glycerin, sorbitol) | 10-45 |
| Thickener (eg cellulose derivative) | 0-3 |
| Sequestering agent (eg citrate) | 0.1-0.4 |
| Flavouring agent | 0.5-2 |
| Sweetener | <0.5 |
| Dyestuff | <0.1 |
| Water | Balance |

### EXAMPLES

The invention is illustrated greater detail in the following non-limiting Examples.

### Example 1: Synthesis of N-decylglyceramide

A solution of methyl glycerate (8.18 g, 0.068 mol) and decylamine (13.6 ml, 0.068 mol) in anhydrous methanol (50 ml) was heated at 60°C under nitrogen for 3 h. The solvent was removed on a rotary evaporator and the residue was crystallised from ethyl acetate which gave the pure compound, melting point 82-90°C (15.32 g, 92%).

The compound showed the following characteristics:
IR (nujol): 3270, 2920, 1640, 1470 cm-1.
¹H NMR (200 MHz, CDCl₃): delta 0.89 (t, CH₃), 1.27 (br, CH₂), 1.52 (m, CH₂), 2.78 (t, OH), 3.24 (m, CH₂) 3.88 (dd, CH), 4.15 (m, OH), 6.97 (t, NH).
¹³C NMR (50 MHz, CDCl₃): delta 14.05, 22.62, 26.94, 29.29, 29.42, 29.55, 31.85, 39.23, 64.47, 72.67, 172.59;
MS (CI, isobutane): MH+, 246.

### Example 2: synthesis of N-octylglyceramide

N-octylglyceramide was prepared by the method of Example 1, using methyl glycerate and n-octylamine as starting materials.

### Example 3

### Foam Height

Foam is an important attribute in many consumer products (eg, consumer products). Foam is one of the dominant factors that determines the commercial value of products such as shampoo, soap, shower gel or dishwashing liquids. Also, acceptability of many consumer products is closely related to the quality and texture of the foam they produce (psychological aspect).

Since most foaming data on surfactants is typically obtained by the Ross-Miles method (Ross J and Miles G D, Am Soc for Testing Material Method D1173-53 Philadelphia, PA, (1953); Oil & Soap (1958) 62 : 1260) the foaming and foam enhancement ability of the surfactants of the invention was also assessed using this method.

In the Ross-Miles method, 200 ml of a solution of surfactant contained in a pipette of specified dimensions with a 2.9 mm internal diameter orifice is allowed to fall 90 cm onto 50 ml of the same solution contained in a cylindrical vessel maintained at a given temperature (often 60°C) by means of a water jacket. the height of the foam produced in the cylindrical vessel is read immediately after all the solution has run out of the pipette (initial foam height) and then again after a given amount of time (generally, 5 min).

Using this method, the foam production (measured initially) and foam stability (the height after 10 minutes) were measured for surfactant systems with and without an N-alkylglyceramide (C₈), the other surfactant being sodium dodecyl sulphate (SDS or C₁₂ PAS). All measurements were carried out at 45°C in water with 120 ppm hardness.

The results are set forth in Table 6 below.

**Table 6:**

| foam stability of PAS/C₈ glyceramide systems | | | |
|---|---|---|---|
| Surfactant system (weight %) | | Foam height (mm) | |
| C₁₂ PAS | C₈ glyceramide | Initial | After 10 minutes |
| 100 | 0 | 145 | 78 |
| | | | |
| 90 | 10 | 165 | 108 |
| | | | |
| 75 | 25 | 158 | 134 |
| | | | |
| 50 | 50 | 169 | 151 |
| | | | |
| 25 | 75 | 172 | 153 |
| | | | |
| 10 | 90 | 155 | 127 |

It can be clearly observed from the numbers above that, when used in sufficient amounts, the N-alkylglyceramide compounds of the invention, clearly enhanced foam stability. Thus, though a 90:10 mixture of PAS/glyceramide showed a foam height of about 108 after 10 minutes, by the time 50:50 mixtures and 25:75 mixtures (all percentages by weight) were used, foam height after 10 minutes was at 151-153 mm.

Thus, the N-alkylglyceramide of the invention enhances foam stability when used as a cosurfactant, particularly when comprising 25 to 90% the surfactant mixture, more preferably, when comprising 40 to 80% by weight of the mixture.

### Example 4: foam enhancement of alkylbenzene sulphonate

This Example demonstrates that the N-alkylglyceramides of the present invention are also valuable when used in small amounts, to enhance or boost the foam of anionic surfactant in the presence of oily soil which acts as a foam suppressant. The anionic surfactant used was sodium dodecyl benzene sulphonate (C₁₂ LAS).

Solutions were prepared at 0.005 molar total surfactant in 1.2 g/litre sodium tripolyphosphate, 0.4 g/litre sodium carbonate and 0.002 molar calcium chloride; this electrolyte was chosen as a model for handwash conditions used in some overseas countries. After equilibration at 25°C, the solutions were clear and colourless.

Foamabilities were measured by the Ross-Miles test described above, at 25°C, in the presence and in the absence of a foam suppressant.

The foam suppressant used was artificial sebum. It was dispersed at a concentration of 1 g/litre in 0.5 litre of surfactant/electrolyte solution at 60°C using a high-speed shear mixer, the solution then being cooled to 25°C before foam testing. The results were as follows:

| Surfactant system (weight %) | | Foam height (mm) | |
|---|---|---|---|
| LAS | C₁₀ glyceramide | Without antifoam | With antifoam |
| 100 | 0 | 137 | 64 |
| | | | |
| 96.34 | 3.66 | 137 | 89 |
| | | | |
| 92.67 | 7.33 | 139 | 108 |

## Claims

1. A compound which is an N-alkylglyceramide or N-alkenylglyceramide of the formula I: 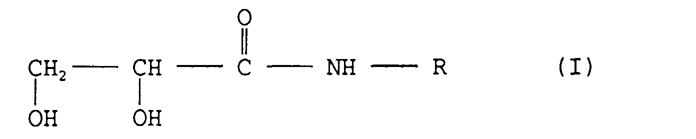 wherein R is a straight- or branched chain alkyl or alkenyl group having from 8 to 24 carbon atoms.

2. A compound as claimed in claim 1, wherein R represents a straight- or branched chain alkyl group having from 8 to 16 carbon atoms.

3. A compound as claimed in claim 1, wherein R represents a linear C₈ or C₁₀ alkyl group.

4. A process for the preparation of a compound as claimed in claim 1, which comprises reacting a C₁₋₄ alkyl ester of glyceric acid with an amine of the formula RNH₂.

5. Use of a compound as claimed in claim 1 as a foaming surfactant and/or foam-enhancing agent in a foaming surface-active composition.

6. A detergent composition comprising a surface-active and foaming and/or foam-enhancing amount of a compound as claimed in claim 1.

7. A detergent composition comprising from 5 to 70 wt% of a surfactant system comprising a compound as claimed in claim 1.

8. A detergent composition as claimed in claim 7, containing a surfactant system comprising from 5 to 95 wt% of a compound as claimed in claim 1 and from 5 to 95 wt% of one or more other surfactants selected from soap, anionic surfactants, nonionic surfactants, cationic surfactants, amphoteric surfactant and zwitterionic surfactants.

9. A detergent composition as claimed in claim 8, wherein the surfactant system comprises from 25 to 90 wt% of a compound as claimed in claim 1 and from 10 to 75 wt% of other surfactant.

10. A detergent composition as claimed in claim 8, wherein the other surfactant comprises an anionic sulphate or sulphonate type detergent.

11. A detergent composition as claimed in claim 10, wherein the other surfactant comprises a primary alcohol sulphate or an alkylbenzene sulphonate.

12. A personal product composition containing a surface-active and foaming and/or foam-enhancing amount of a compound as claimed in claim 1, wherein the compositions is a toilet bar, a facial and/or body cleanser, a shampoo, a conditioner, a conditioning shampoo, a shower gel, a cosmetic composition, an antiperspirant or deodorant composition, a shaving preparation, or a toothpaste composition.

## Patentansprüche

1. Verbindung, nämlich ein N-Alkylglyceramid oder N-Alkenylglyceramid der Formel I: 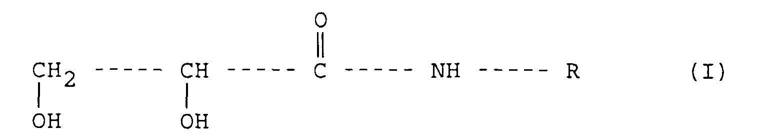 worin R eine gerad- oder verzweigtkettige Alkyl- oder Alkenylgruppe mit 8 bis 24 Kohlenstoffatomen darstellt.

2. Verbindung nach Anspruch 1, worin R eine gerad- oder verzweigtkettige Alkylgruppe mit 8 bis 16 Kohlenstoffatomen wiedergibt.

3. Verbindung nach Anspruch 1, worin R eine lineare C₈- oder C₁₀ Alkylgruppe wiedergibt.

4. Verfahren zur Herstellung einer Verbindung nach Anspruch 1, umfassend Umsetzen eines C₁₋₄-Alkylesters von Glycerinsäure mit einem Amin der Formel RNH₂.

5. Verwendung einer Verbindung nach Anspruch 1 als Schaumtensid und/oder schaumverstärkendes Mittel in einer grenzflächenaktiven Schaumzusammensetzung.

6. Waschmittelzusammensetzung, umfassend eine grenzflächenaktive und schäumende und/oder schaumverstärkende Menge einer Verbindung nach Anspruch 1.

7. Waschmittelzusammensetzung, umfassend 5 bis 70 Gew.-% eines Tensidsystems, umfassend eine Verbindung nach Anspruch 1.

8. Waschmittelzusammensetzung nach Anspruch 7, enthaltend ein Tensidsystem, umfassend 5 bis 95 Gew.-% einer Verbindung nach Anspruch 1 und 5 bis 95 Gew.-% eines oder mehrerer anderer Tenside, ausgewählt aus Seife, anionischen Tensiden, nichtionischen Tensiden, kationischen Tensiden, amphoterem Tensid und zwitterionischen Tensiden.

9. Waschmittelzusammensetzung nach Anspruch 8, worin das Tensidsystem 25 bis 90 Gew.-% einer Verbindung nach Anspruch 1 und 10 bis 75 Gew.-% anderes Tensid umfaßt.

10. Waschmittelzusammensetzung nach Anspruch 8, worin das andere Tensid ein Waschmittel vom anionischen Sulfat- oder Sulfonattyp umfaßt.

11. Waschmittelzusammensetzung nach Anspruch 10, worin das andere Tensid ein primäres Alkoholsulfat oder ein Alkylbenzolsulfonat umfaßt.

12. Körperpflegeprodukt, enthaltend eine grenzflächenaktive und schäumende und/oder schaumverstärkende Menge einer Verbindung nach Anspruch 1, wobei das Mittel einen Toilettenseifenriegel, ein Gesicht- und/oder Körperreinigungsmittel, ein Shampoo, ein Konditionierungsmittel, ein Konditionierungsshampoo, ein Duschgel, ein kosmetisches Mittel, ein Antischweißmittel oder ein Desodorans, eine Rasierzubereitung oder eine Zahnpasta darstellt.

## Revendications

1. Composé qui est un N-alkylglycéramide ou un N-alcénylglycéramide de formule I : 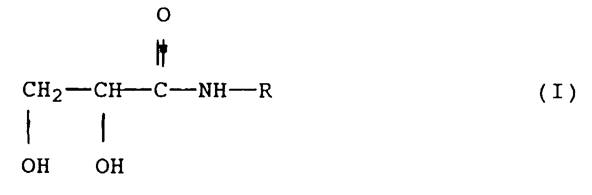 dans laquelle R est un groupe alkyle ou alcényle à chaîne droite ou ramifiée contenant de 8 à 24 atomes de carbone.

2. Composé selon la revendication 1, dans lequel R représente un groupe alkyle à chaîne droite ou ramifié ayant de 8 à 16 atomes de carbone.

3. Composé selon la revendication 1, dans lequel R représente un groupe alkyle linéaire en C₈ ou C₁₀.

4. Procédé de préparation d'un composé selon la revendication 1, qui consiste à faire réagir un ester alkylique en C₁₋₄ de l'acide glycérique avec une amine de formule RNH₂.

5. Utilisation d'un composé selon la revendication 1, comme tensioactif moussant et/ou agent de renforcement de la mousse dans une composition tensioactive moussante.

6. Composition détergente comprenant une quantité tensioactive et de moussage et/ou de renforcement de la mousse d'un composé selon la revendication 1.

7. Composition détergente comprenant de 5 à 70% en poids d'un système tensioactif comprenant un composé selon la revendication 1.

8. Composition détergente selon la revendication 7, contenant un système tensioactif comprenant de 5 à 95% en poids d'un composé selon la revendication 1 et de 5 à 95% en poids d'un (ou plusieurs) autre tensioactif choisi parmi le savon, les tensioactifs anioniques, les tensioactifs non ioniques, les tensioactifs cationiques, les tensioactifs amphotères et les tensioactifs zwitterioniques.

9. Composition détergente selon la revendication 8, dans laquelle le système tensioactif comprend de 25 à 90% en poids d'un composé selon la revendication 1 et de 10 à 75% en poids d'un autre tensioactif.

10. Composition détergente selon la revendication 8, dans laquelle l'autre tensioactif comprend un détergent du type sulfate ou sulfonate anionique.

11. Composition détergente selon la revendication 10, dans laquelle l'autre tensioactif comprend un alcool-sulfate primaire ou un alkylbenzène-sulfonate.

12. Composition de produit personnel contenant une quantité tensioactive et de moussage et/ou de renforcement de la mousse d'un composé selon la revendication 1, dans laquelle la composition est un pain de toilette, un nettoyant pour le visage et/ou le corps, un shampooing, un conditionneur, un shampooing de conditionnement, un gel de douche, une composition cosmétique, une composition anti-perspirante ou déodorante, une préparation pour le rasage ou une composition dentifrice.
